# EUROPÄISCHE PATENTSCHRIFT

(11) **EP 2 501 246 B1**
(45) Veröffentlichungstag und Bekanntmachung des Hinweises auf die Patenterteilung: **20.09.2017**
(21) Anmeldenummer: 10773009.5
(22) Anmeldetag: 14.10.2010
(51) Int. Cl.: A41D 13/12, F41H 1/02, G01L 5/00, H01B 1/20

(54) **BEKLEIDUNGSSTÜCK FÜR EINEN MENSCHLICHEN KÖRPER**
PIECE OF CLOTHING FOR A HUMAN BODY
VÊTEMENT POUR UN CORPS HUMAIN

(30) Priorität: 19.11.2009 DE 102009046861
(43) Veröffentlichungstag der Anmeldung: 26.09.2012
(73) Patentinhaber: Hexonia GmbH, 41334 Nettetal (DE)
(72) Erfinder: HEXELS, Gerd, 41334 Nettetal (DE)
(74) Vertreter: Lorenz, Markus
(86) Internationale Anmeldenummer: PCT/EP2010/065441
(87) Internationale Veröffentlichungsnummer: WO 2011/061019

(56) Entgegenhaltungen:
- WO-A2-2008/118296
- US-A- 4 824 107
- US-A1- 2006 053 534
- US-A1- 2009 112 078
- US-A1- 2009 235 761
- US-B1- 6 315 009
- US-B1- 6 349 201

## Beschreibung

Die Erfindung betrifft ein Bekleidungsstück für einen menschlichen Körper, mit Sensoren, die externe Einwirkungen detektieren und ein Signal erzeugen, welches an eine Auswerteeinheit weiterleitbar ist.

Ein gattungsgemäßes Bekleidungsstück ist aus der US 6,315,009 B1 bekannt.

Insbesondere bei militärischen Einsätzen, jedoch auch bei Polizeieinsätzen sind Menschen der Gefahr ausgesetzt, verletzt zu werden, gegebenenfalls lebensgefährlich oder sogar tödlich. Dabei kann es von besonderer Bedeutung sein, wenn eine zentrale Leitstelle oder dergleichen frühzeitig darüber informiert wird, dass eine im Einsatz befindliche Person verletzt wurde. Des weiteren kann es für eine Rettung der Person von Bedeutung sein, wenn die Rettungskräfte, beispielsweise Sanitäter, bereits vor deren Eintreffen bei der verletzten Person darüber informiert sind, welche Art die Verletzung ist und gegebenenfalls wie schwer die Verletzung ist.

Die aus der US 6,315,009 B1 bekannte Lösung sieht vor, dass ein Bekleidungsstück, beispielsweise in Form eines Shirts, mit optischen Sensoren versehen ist. Diese können als Glasfaserleiter oder Lichtwellenleiter auf Basis von Quarzkunststoff oder Silikon aufgebaut sein. Dabei ist vorgesehen, dass ein Signal erzeugt wird, wenn ein Projektil die optische Faser durchschlägt.

Durch das Signal wird eine Auswerteeinheit darüber informiert wird, dass der Lichtwellenleiter unterbrochen ist.

Zum Stand der Technik wird auch auf die US 4,824,107 A verwiesen. Die US 4,824,107 A zeigt ein Bekleidungsstück für einen menschlichen Körper mit Sensoren, die externe Einwirkungen detektieren und ein Signal erzeugen, welches an eine Auswerteeinheit weiterleitbar ist. Die Sensoren weisen eine piezoelektrische Polymerfolie auf, welche geeignet ist, eine mechanische Einwirkung zu erkennen.

Zum weiteren Stand der Technik wird ferner auf die US 6,687,523 B1, die WO 2008/118296, die US 2006/053534, die US 2009/235761, die US 6,349,201 B1 und die US 2009/112078 A1 verwiesen.

Von Nachteil bei dem bekannten Stand der Technik, insbesondere bei der US 6,315,009 B1 und der US 6,687,523 B1, ist es, dass lediglich ein Signal erzeugt werden kann, welches darüber informiert, dass der Lichtwellenleiter durchbrochen ist. Das Signal enthält keine Information darüber, wo ein Projektil oder eine Stichwaffe in einen menschlichen Körper eingedrungen ist. Die Rettungskräfte treffen daher entsprechend unvorbereitet auf die verletzte Person. Ferner ist es von Nachteil, dass eine Verletzung durch einen Schlag, welcher gegebenenfalls auch zu schweren Verletzungen (Trauma) bis hin zum Tod der getroffenen Person führen kann, nicht detektiert wird.

Der vorliegenden Erfindung liegt daher die Aufgabe zugrunde, ein Bekleidungsstück für einen menschlichen Körper zu schaffen, welches es ermöglicht, Verletzungen am menschlichen Körper, insbesondere durch Stich- oder Schusswaffen, zuverlässig zu erkennen und welches es ermöglicht, die Verletzung möglichst genau einem gegebenenfalls betroffenen lebenswichtigen Organ zuzuordnen.

Diese Aufgabe wird erfindungsgemäß durch Anspruch 1 gelöst.

Es wird bei der nachfolgenden Beschreibung davon ausgegangen, dass das Bekleidungsstück in der vorgesehenen Weise korrekt getragen wird.

Dadurch, dass die Sensoren, mit denen das Bekleidungsstück versehen ist, als elektrische Schaltkreise und/ oder als Elemente ausgebildet sind, deren Sensoreneigenschaft auf dem Piezoeffekt beruht und die Sensoren wenigstens in einer Zone des Bekleidungsstücks angeordnet sind, die an lebenswichtige Organe angrenzt, lässt sich, verglichen mit dem Stand der Technik, verhältnismäßig präzise feststellen, an welcher Stelle des Körpers die das Bekleidungsstück tragende Person getroffen wurde. Gemäß dem Stand der Technik konnte lediglich festgestellt werden, dass ein Lichtwellenleiter unterbrochen ist, eine genauere Zuordnung war nicht möglich.

Von Vorteil bei der erfindungsgemäßen Lösung ist zudem, dass auch ein Mehrfachbeschuss erkannt bzw. aufgezeichnet werden kann.

Bei einer Ausgestaltung der Sensoren als elektrische Schaltkreise bzw. elektrische Leiterbahnen lässt sich mit bekannten technischen Mitteln feststellen, an welcher Position der Schaltkreis bzw. die Leiterbahn unterbrochen ist. Durch die Kenntnis der Unterbrechungsstelle an dem Bekleidungsstück können Rückschlüsse auf den Bereich des menschlichen Körpers, der von einem Projektil oder einer Stichwaffe getroffen wurde, geschlossen werden. Alternativ oder ergänzend können auch eine Vielzahl von kleinen elektrischen Schaltkreisen bzw. Netzwerken eingesetzt werden, so dass bei einem Ausfall eines Schaltkreises aufgrund dessen Position auf dem Bekleidungsstück Rückschlüsse auf die Verletzungsstelle möglich sind.

Von Vorteil ist es, wenn die elektrischen Schaltkreise in Wellen- oder Kurvenform verlaufende Leiterbahnen aufweisen. Die Erfinder haben erkannt, dass es vorteilhaft ist, die Leiterbahnen so elastisch anzuordnen, dass diese durch eine Zugbewegung des Bekleidungsstücks nicht abgerissen werden. Besonders vorteilhaft lässt sich eine derartige Anordnung erreichen, wenn die Leiterbahnen in Wellen- oder Kurvenform verlaufen, so dass eine Zug- bzw. Dehnbewegung der Leiterbahnen ermöglicht wird.

Auch bei einer Ausbildung der Leiterbahnen durch ein nachfolgend näher beschriebenes, elektrisch leitendes Polymer hat es sich als vorteilhaft herausgestellt, wenn die Leiterbahnen in Wellen oder Kurvenform verlaufen. Bei einem elektrisch leitenden Polymer handelt es sich im Rahmen der Erfindung um eine spezifische Gestaltung eines elektrischen Schaltkreises, so dass die bezüglich des elektrischen Schaltkreises beschriebenen Merkmale immer auch für das elektrisch leitende Polymer gelten.

Besonders vorteilhaft ist es, wenn die elektrischen Schaltkreise bzw. die Leiterbahnen direkt auf das Bekleidungsstück aufgedruckt bzw. gedruckt sind. Dabei kann auch vorgesehen sein, dass die elektrischen Schaltkreise bzw. die Leiterbahnen auf eine Textilie gedruckt werden, welche anschließend mit dem Bekleidungsstück in bekannter Weise verbunden wird.

Die Sensoren, die als Elemente ausgebildet sind, deren Sensoreigenschaft auf dem Piezoeffekt beruht, werden nachfolgend kurz als Piezoelemente bezeichnet.

Ein Piezoelement ist ein Bauteil, das den sogenannten Piezoeffekt ausnützt, um beispielsweise bei Einwirkung einer mechanischen Kraft (z. B. Druck) eine elektrische Spannung zu erzeugen. Piezoelemente können bestimmte Kristalle (Piezokristalle) oder piezoelektrische Keramiken, also polykristalline Materialien, sein. Ein besonders geeigneter Werkstoff kann Blei-Zirkonat-Titanat sein.

Die Ausbildung der Sensoren als Piezoelemente hat den Vorteil, dass diese auf Druck ansprechen und es somit auch möglich ist, einen Schlag zu detektieren, der gegen die Person, die das Bekleidungsstück trägt, ausgeführt wird. Es ist somit nicht nur möglich, Schuss- oder Stichverletzungen festzustellen, sondern auch gefährliche Schläge zu erkennen. Eine Ausbildung von Piezoelementen, beispielsweise in Kopfbedeckungen, bei denen Schläge besonders gefährlich sind, oder im Herzbereich, kann besonders geeignet sein. Von Vorteil kann es zudem sein, wenn die Piezoelemente gemeinsam mit Sensoren auf Basis von elektrischen Schaltkreisen eingesetzt werden. Dadurch wird besonders vorteilhaft sowohl eine Stich- oder Schussverletzung als auch eine Verletzung durch einen Schlag erkannt.

Durch die piezoelektrische Ausgestaltung der Sensoren können nicht nur Schläge, sondern auch Druckwellen, Detonationen und dergleichen erkannt werden.

Anstelle von Piezoelementen können auch andere bekannte Drucksensoren eingesetzt werden. Vorliegend soll der Begriff "Piezoelement" in seiner allgemeinsten Bedeutung daher auch beliebige Drucksensoren umfassen.

Von Vorteil ist es, wenn die Sensoren, d. h. die elektrischen Schaltkreise oder die Piezoelemente mehrere Zonen ausbilden, die unterschiedlichen lebenswichtigen Organen zugeordnet sind.

Durch die Ausbildung von mehreren Zonen lässt sich eine ungewünschte Einwirkung auf den menschlichen Körper besonders vorteilhaft erkennen und zuordnen. Bei den lebenswichtigen Organen, denen entsprechende Zonen zugeordnet werden, kann es sich vorzugsweise um das Herz, die Lunge, den Magen, den Darm, die Leber oder die Nieren handeln. Vorzugsweise sind die Zonen sowohl an der Vorderseite als auch an der Rückseite des Bekleidungsstücks ausgebildet. Die Zonen können sich dabei gegenseitig überlappen bzw. ineinander geschachtelt angeordnet sein. Vorteilhaft kann es des weiteren sein, wenn die Zonen insgesamt wenigstens annähernd, vorzugsweise vollständig, das Bekleidungsstück umfassen, so dass das Bekleidungsstück keinen Bereich aufweist, der nicht überwacht wird.

Durch die front- und rückseitige Anwendung des Systems bzw. der Zonen können im Gegensatz zu der aus dem Stand der Technik bekannten Lichtleitertechnik auch Durchschüsse erkannt werden.

Die Zonen können an die anatomischen und organspezifischen Anordnungen in dem menschlichen Körper angepasst sein. Beispielsweise bei einer Ausbildung der Zone durch einen elektrischen Schaltkreis wird, wenn die Zone z. B. durch ein Geschoss zerstört wird, der Schaltkreis unterbrochen. Dadurch kann eine Meldung über einen Regelkreis an eine Auswerteeinheit generiert werden. Gegebenenfalls kann über Funk fortwährend eine Information über den Zustand des Trägers des Bekleidungsstücks an eine Überwachungsstation übermittelt werden.

Zur Übertragung des Signals kann eine Funkeinheit in das Bekleidungsstück integriert sein oder eine Verbindung zu einer externen Funkeinheit vorgesehen sein. Ferner kann eine Schnittstelle, vorzugsweise eine USB-Schnittstelle, vorgesehen sein, um die Sensoren und/ oder die Auswerteeinheit auszulesen.

Das Signal kann von der Funkeinheit permanent, in Intervallen oder wenn die Sensoren eine externe Einwirkung detektiert haben, gesendet werden.

Von Vorteil ist es, wenn Vitalsensoren vorgesehen sind, welche vorzugsweise den Herzschlag bzw. Puls und/oder die Atemfrequenz und/oder die Sauerstoffsättigung im Blut und/oder die Körpertemperatur und/oder den Blutdruck erfassen. Diese Informationen können ebenfalls an eine Auswerteeinheit bzw. an eine Überwachungseinheit weitergeleitet werden. Dadurch kann im Falle einer Detektion eines Schlags oder einer Stich- oder Schussverletzung schnell erkannt werden, ob die externe Einwirkung auf den menschlichen Körper lebensgefährlich ist. Die notwendigen Maßnahmen können dann entsprechend koordiniert werden. Darüber hinaus können durch eine Erfassung der Vitalfunktionen Fehlmeldungen reduziert werden. Gegebenenfalls kann vorgesehen sein, dass die Ermittlung der Vitalfunktionen nicht permanent erfolgt, sondern durch einen Funkbefehl ausgelöst wird. Beispielsweise könnte durch einen Funkbefehl eine Blutdruckmessung ausgelöst werden.

Von Vorteil ist insbesondere eine Temperaturmessung, da im Regelfall die Temperatur eines menschlichen Körpers ansteigt, wenn dieser durch einen Treffer verletzt wird. Die Detektion eines Treffers in Verbindung mit einem Ansteigen der Temperatur kann somit hilfreich sein, auf eine möglicherweise lebensbedrohliche Situation hinzuweisen.

Zur Ermittlung des Herzschlags kann beispielsweise vorgesehen sein, dass in das Bekleidungsstück in dem Bereich, der an das Herz angrenzt, ein entsprechender Vitalsensor integriert ist. Ferner kann beispielsweise zur Ermittlung der Atemfrequenz im Bereich der Lunge ein entsprechender Vitalsensor in dem Bekleidungsstück integriert sein.

Die Sensoren auf Basis der Piezoelemente können in beliebiger Art und Weise mit dem Bekleidungsstück verbunden sein. Besonders bevorzugt ist eine Ausbildung der Piezoelemente als Piezofolie. Diese kann in einfacher Weise mit dem Bekleidungsstück verbunden werden.

Von Vorteil kann es sein, wenn die Sensoren auf Basis der elektrischen Schaltkreise als gedruckte Schaltungen ausgebildet sind. Die Schaltungen können dabei auf Folien oder auf andere biegsame Materialien gedruckt werden.

Die Sensoren, insbesondere die elektrischen Schaltkreise, können auch in das Bekleidungsstück eingestickt oder eingewebt sein.

Insofern vorgesehen ist, die Piezoelemente gemeinsam mit elektrischen Schaltkreisen einzusetzen, kann vorgesehen sein, dass die eingesetzten Sensorentypen in zwei Lagen vorzugsweise übereinander im Bekleidungsstück aufgebracht bzw. integriert sind. Auch hierfür eignet es sich, die Sensorentypen jeweils in Folien zu integrieren. Gegebenenfalls kann vorgesehen sein, dass das Bekleidungsstück nur an den Stellen, an denen dieses an schlagempfindliche Bereiche des Körpers angrenzt, mit zwei Sensorentypen versehen ist.

Das erfindungsgemäße Bekleidungsstück kann auf beliebige Art und Weise, vorzugsweise für einen menschlichen Oberkörper, ausgebildet sein. Das Bekleidungsstück kann dabei als Unterwäsche, als Shirt bzw. T-Shirt, als Overall, als herkömmliches Oberbekleidungsstück, als Jacke, als Kopfbedeckung oder dergleichen ausgebildet sein. Grundsätzlich möglich ist auch eine Ausbildung des Bekleidungsstücks als Hose. Von Vorteil ist es, wenn das Bekleidungsstück atmungsaktiv und/oder winddicht und/oder wasserdicht und/oder wasserdampfdurchlässig ist. Besonders bevorzugt ist es, wenn das Bekleidungsstück alle genannten Eigenschaften aufweist.

Von Vorteil ist es, wenn die Sensoren in ihrer Ausbildung als Elemente, deren Sensoreigenschaft auf dem Piezoeffekt beruht, als Polymer ausgebildet sind. Dabei ist es besonders zu bevorzugen, wenn es sich bei dem Polymer um ein Polyvinylidenfluorid (PVDF) handelt. Vorzugsweise kann das Polyvinylidenfluorid durch Polarisation piezoelektrische Eigenschaften aufweisen. Das Polyvinylidenfluorid ist vorzugsweise als Folie oder als Schicht ausgebildet.

Von Vorteil ist es, wenn das Polyvinylidenfluorid in einer Wabenstruktur auf das Bekleidungsstück aufgebracht wird bzw. eine Wabenstruktur aufweist. Dadurch bleiben die positiven bekleidungsphysiologischen Eigenschaften des Bekleidungsstücks erhalten. Ein durch einen Schlag oder einen ballistischen Einschlag hervorgerufener Druck auf das Bekleidungsstück beaufschlagt immer eine größere Fläche als ein Punkt der Wabenstruktur aufweist. Ein Schlag bzw. ein ballistischer Einschlag wird somit nicht zwischen eine Wabenstruktur passen und kann somit durch die piezoelektrischen Eigenschaften des Polyvinylidenfluorid erkannt werden.

In einer Ausgestaltung der Erfindung kann vorgesehen sein, dass das Polyvinylidenfluorid wenigstens in einer Zone des Bekleidungsstücks angeordnet ist. Vorzugsweise ist das Polyvinylidenfluorid an der Vorderseite und/oder der Rückseite des Bekleidungsstücks ausgebildet. Vorzugsweise ist das Bekleidungsstück vollständig bzw. wenigstens annähernd vollständig mit einer Schicht des Polyvinylidenfluorid, vorzugsweise mit einer Schicht, die durch eine Wabenstruktur gebildet ist, versehen.

Auf die Schicht aus dem Polyvinylidenfluorid (PVDF) kann eine Leiterstruktur bzw. Leiterbahnen aufgebracht werden. Die Leiterstruktur bzw. die Leiterbahnen können somit die Impulse des Polyvinylidenfluorids weiterleiten bzw. aufnehmen. Zur Ausbildung der Leiterstruktur bzw. Leiterbahnen sind aus dem Stand der Technik verschiedene Möglichkeiten bekannt. Beispielsweise kann die Leiterstruktur bzw. können die Leiterbahnen durch eine Belichtungs- und Ätztechnologie unter Verwendung von Kupfer aufgebracht werden.

Die Leiterstruktur bzw. die Leiterbahnen können mit einer atmungsaktiven Beschichtung und/oder einer entsprechenden Folie überzogen werden, insbesondere um eine Oxidation zu vermeiden, insbesondere wenn es sich bei der Leiterstruktur bzw. den Leiterbahnen um Kupferbahnen handelt.

Die Ausbildung von Leiterbahnen und Leiterstrukturen, kann auf beliebigen Substraten, vorzugsweise in einer Mäandertechnik erfolgen.

Von Vorteil ist es des weiteren, wenn die Leiterbahnen bzw. die Leiterstruktur Verbindungsstellen zur Anbindung weiterer Sensoren, insbesondere von Vitalsensoren, wie sie vorstehend bereits dargestellt wurden, aufweisen. Dadurch können an beliebigen Stellen Messungen, beispielsweise des Pulses oder anderer Vitalfunktionen, erfolgen.

Von Vorteil ist es, wenn die Energieversorgung des Systems durch Folienbatterien erfolgt, die vorzugsweise aufgedruckt sind.

Eine besonders vorteilhafte Ausbildung der Leiterbahnen und der Leiterstruktur bzw. allgemein eine vorteilhafte Möglichkeit, um Impulse zu übertragen, besteht darin, auf das Polymer mit den piezoelektrischen Eigenschaften ein weiteres elektrisch leitfähiges Polymer, beispielsweise auf Basis von Polyethylendioxythiopen (PEDOT) aufzubringen. Vorzugsweise wird das Polyethylendioxythiopen dabei auf eine Schicht bzw. eine Fläche bzw. eine Folie, die durch ein Polyvinylidenfluorid gebildet ist, aufgebracht. Bei einem Polyethylendioxythiopen handelt es sich um ein elektrisch leitfähiges Polymer, das im Stand der Technik für flexible Displays, flexible Solarzellen und sogenannte organische LEDs verwendet wird.

Das elektrisch leitende Polymer, das auf das Polymer mit den piezoelektrischen Eigenschaften aufgebracht ist, kann die Zerstörung bzw. Beschädigung des Systems, beispielsweise durch einen Beschuss, registrieren. Das elektrisch leitende Polymer kann dabei vorzugsweise die Funktion des ballistischen Leiters übernehmen, d. h. die Funktion, die vorstehend bereits bezüglich des elektrischen Schaltkreises beschrieben wurde. Das elektrisch leitfähige Polymer kann somit vorzugsweise die Funktionen erfüllen, die der elektrische Schaltkreis erfüllt. Die vorstehend beschriebenen Merkmale bezüglich des elektrischen Schaltkreises gelten somit auch für das elektrisch leitende Polymer, insbesondere das Polyethylendioxythiopen, insbesondere wenn dieses in Form von Leiterbahnen bzw. einer Leiterstruktur vorliegt.

Erfindungsgemäß kann eine Schaltung vorgesehen sein, welche zum Einen über die elektrisch leitenden Polymere Impulse des Polyvinylidenfluorids erkennt und zum Anderen eine Zerstörung der durch das elektrisch leitende Polymer, vorzugsweise des Polyethylendioxythiopen, gebildeten Leiterbahnen erkennt. Dies kann vorzugsweise dadurch erfolgen, dass einerseits eine Spannung aufgebaut wird, um die Impulse des piezoelektrischen Polymers aufzunehmen und andererseits eine Spannung angelegt wird, um zu erkennen, ob die Leiterbahnen unterbrochen bzw. zerstört sind. Vorzugsweise kann eine Polyesterstrickware oder dergleichen netzartig mit dem Polyvinylidenfluorid als Piezo-Sensor beschichtet sein. Darüber oder darunter können dann Leiterbahnen, vorzugsweise aus einem Polyethylendioxythiopen, vorzugsweise mäanderförmig, aufgedruckt sein.
Eine besonders vorteilhafte Ausgestaltung des Bekleidungsstücks besteht darin, dieses als soft- oder hartballistische Schutzweste auszubilden. Derartige soft- oder hartballistische Schutzwesten sind aus dem allgemeinen Stand der Technik hinlänglich bekannt. Derartige Schutzwesten werden im Regelfall von gefährdeten Personen, beispielsweise von Soldaten oder von Polizeibeamten, getragen. Gerade in diesem Fall ist es von besonderem Vorteil, wenn externe Einwirkungen auf den menschlichen Körper erkannt werden. Die Schutzweste, die auch als Panzerweste bezeichnet wird, kann an ihrer Vorder- und/oder Rückseite soft- und/ oder hartballistische Einlagen aufweisen. Von Vorteil kann es dabei sein, wenn lediglich die ballistischen Pakete (softballistisch oder hartballistisch) mit Sensoren, beispielsweise einer Folienschaltkreismembran, überzogen sind. Bei einer Zerstörung der ballistischen Schutzpakete wird damit gleichzeitig eine Signalwirkung generiert. Auch in diesem Fall können innerhalb des Systems Vitalfunktionen abgetastet und Biosensoren integriert sein.

Von Vorteil ist bei einer Ausgestaltung des Bekleidungsstücks als Schutzweste, wenn die Sensoren auf der dem Körper zugewandten Seite der Schutzweste angeordnet sind. Die Anordnung kann dabei auf der dem Körper zugewandten Seite der ballistischen Schutzpakete der Schutzweste erfolgen. Von Vorteil ist es, wenn die Sensoren auf der Außenhülle der soft- oder hartballistischen Schutzpakete aufgebracht bzw. integriert sind. Von Vorteil kann es zudem sein, wenn die Sensoren, insbesondere in einer Ausgestaltung als elektrische Schaltkreise, auf einer Schutzfolie, vorzugsweise einer UV-Schutzfolie der ballistischen Schutzpakete angeordnet sind. Im Regelfall weisen insbesondere die softballistischen Schutzpakete ohnehin eine Schutzfolie auf, welche diese gegen ungewünschte Beeinträchtigungen, beispielsweise durch UV-Strahlung oder dem Eindringen von Wasser, schützen soll. Auf diese Schutzfolien können dann die Sensoren aufgebracht sein.

Von Vorteil ist es, wenn die Schutzweste mit einer Recheneinheit zur Auswertung der Signale versehen ist oder alternativ eine Verbindung zu einer Rechnereinheit besteht.

Gut ausgerüstetes militärisches Personal führt mittlerweile Schutzwesten mit sich, in die bereits entsprechende Rechnereinheiten integriert sind.

Vorteilhafte Ausgestaltungen und Weiterbildungen der Erfindung ergeben sich aus den weiteren abhängigen Ansprüchen. Nachfolgend ist ein Ausführungsbeispiel prinzipmäßig dargestellt.

Es zeigt:
- Fig. 1: eine Draufsicht auf ein erfindungsgemäßes Bekleidungsstück mit mehreren exemplarisch dargestellten Zonen, die von prinzipmäßig dargestellten Sensoren überwacht sind;
- Fig. 2: ein erfindungsgemäßes Bekleidungsstück, welches vollständig mit prinzipmäßig dargestellten Sensoren versehen ist;
- Fig. 3: ein erfindungsgemäßes Bekleidungsstück mit einer prinzipmäßigen Darstellung der Anbindung der überwachten Zonen an eine Funkeinheit und eine weitere Schnittstelle;
- Fig. 4: ein erfindungsgemäßes Bekleidungsstück mit einer prinzipmäßigen Darstellung eines Vitalsensors im Bereich eines Herzens eines Trägers des Bekleidungsstücks;
- Fig. 5: eine prinzipmäßige Darstellung einer Schutzweste;
- Fig. 6: eine prinzipmäßige Darstellung eines softballistischen Schutzpakets;
- Fig. 7: eine prinzipmäßige Darstellung eines hartballistischen Schutzpakets.

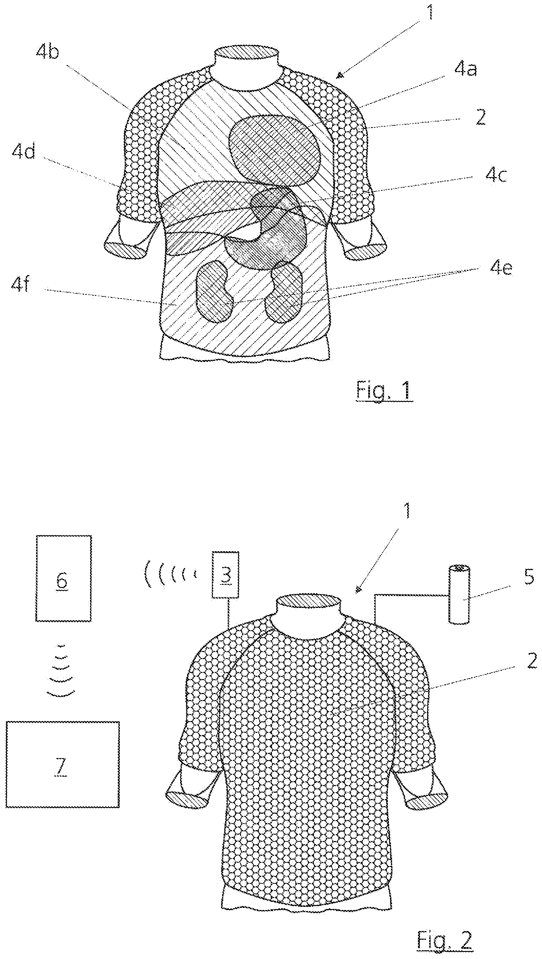

In den Figuren 1 bis 4 ist das erfindungsgemäße Bekleidungsstück anhand eines Shirts dargestellt. Der grundsätzliche Aufbau von derartigen Bekleidungsstücken ist aus dem allgemeinen Stand der Technik hinlänglich bekannt.

Im Ausführungsbeispiel ist vorgesehen, dass das Shirt winddicht, atmungsaktiv, wasserdampfdurchlässig und wasserdicht ist.

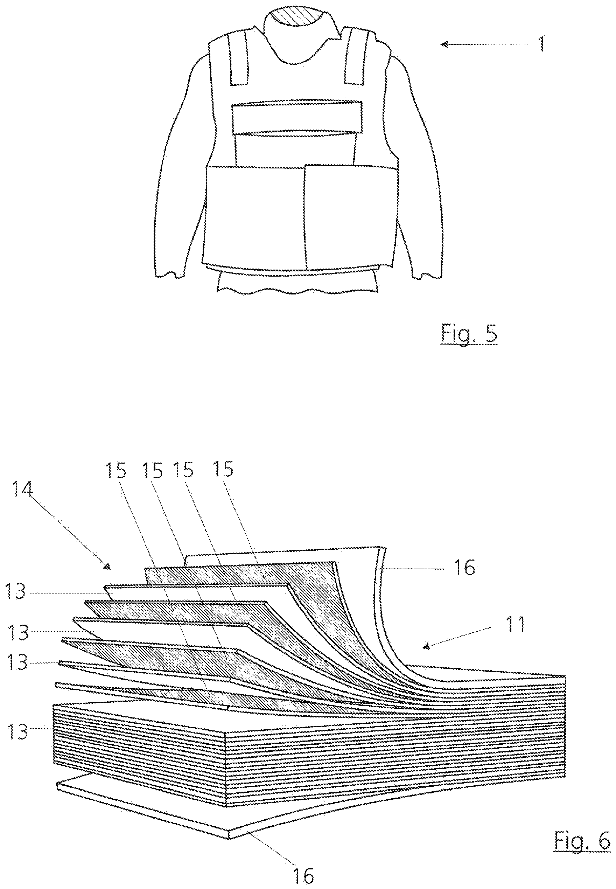

In Fig. 5 ist das Bekleidungsstück 1 als Schutzweste ausgebildet.

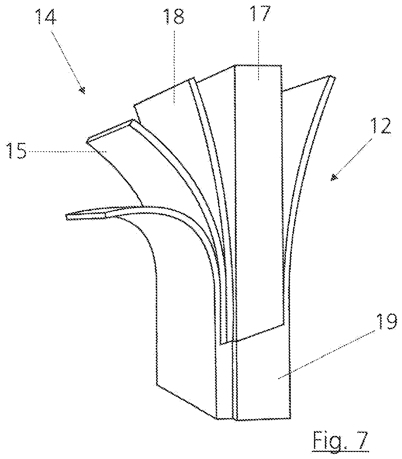

In den Figuren 6 bis 7 ist ein Schutzpaket in einer Ausgestaltung als softballistisches Schutzpaket (Fig. 6) und in einer Ausgestaltung als hartballistisches Schutzpaket (Fig. 7) dargestellt. Derartige Schutzpakete sind aus dem allgemeinen Stand der Technik grundsätzlich bekannt, weshalb nur auf die spezifische Ausgestaltung des Schutzpakets näher eingegangen wird.

Die Merkmale, die hinsichtlich der Figuren 1 bis 4 und 5 bis 7 nachfolgend beschrieben werden, sind grundsätzlich austauschbar, d. h. die nachfolgend beschriebenen Merkmale sind bei beiden Ausführungsformen (Shirt und Schutzweste) einsetzbar.

Wie aus Fig. 1 ersichtlich ist, weist das Bekleidungsstück 1 für einen menschlichen Körper, vorliegend für einen menschlichen Oberkörper, Sensoren 2 auf, die externe Einwirkungen detektieren und ein Signal an eine in Fig. 2 prinzipmäßig dargestellte Auswerteeinheit 3 weiterleiten.

Fig. 1 zeigt die Sensoren 2 aus Übersichtsgründen lediglich im Bereich der Ärmel des Bekleidungsstücks 1. Es ist jedoch vorgesehen, dass alle nachfolgend näher erläuterten Zonen 4 mit den Sensoren versehen sind. Lediglich um den Verlauf der unterschiedlichen Zonen 4 darzustellen, wurden diese nicht noch zusätzlich mit Sensoren 2 abgebildet.

Die in den Figuren 1 bis 4 dargestellten Sensoren 2 können in einer ersten Ausführungsform im Ausführungsbeispiel als elektrische Schaltkreise ausgebildet sein. Grundsätzlich könnten die Sensoren 2 auch als beliebiges elektrisches Netzwerk, als druckempfindliche Sensoren oder als Piezoelemente ausgebildet sein.

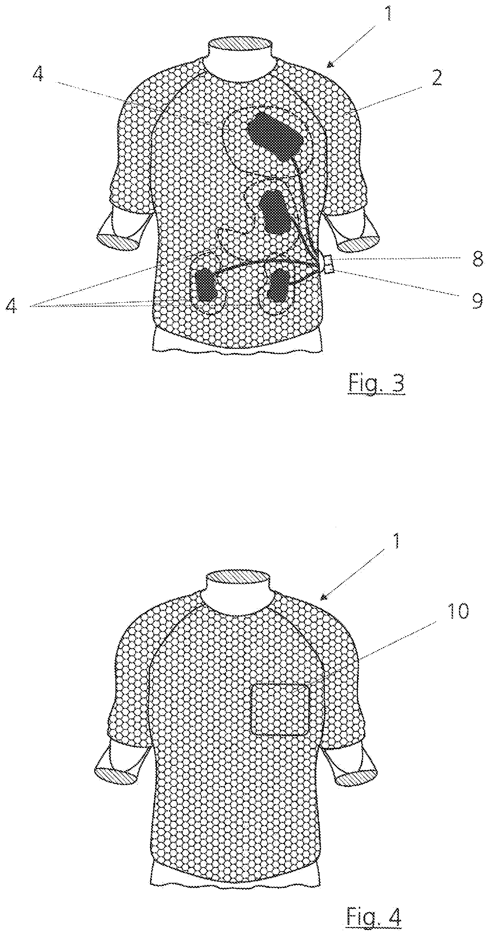

Wie sich aus Fig. 1 und Fig. 3 ergibt, sind die elektrischen Schaltkreise 2 bzw. deren Leiterbahnen in mehreren Zonen 4 ausgebildet, die unterschiedlichen lebenswichtigen Organen zugeordnet sind. In Fig. 3 sind aus Übersichtsgründen lediglich vier Zonen 4 dargestellt. Vorzugsweise sind jedoch auch in dem Ausführungsbeispiel gemäß Fig. 3 die Zonen 4 ausgebildet, die in der Fig. 1 dargestellt sind.

Im Ausführungsbeispiel gemäß Fig. 1 ist eine Zone 4a für das Herz, eine Zone 4b für die beiden Lungenflügel, eine Zone 4c für den Magen, eine Zone 4d für die Leber, zwei Zonen 4e für die Nieren und eine große Zone 4f für den Darm vorgesehen. Die Zonen 4 können sich dabei gegenseitig überlappen.

Fig. 2 zeigt eine Ausgestaltung, bei der die elektrischen Schaltkreise 2 bzw. deren Leiterbahnen das gesamte Bekleidungsstück 1 bedecken. Auf die Ausbildung spezifischer Zonen 4 wurde dabei verzichtet.

In Fig. 2 ist eine prinzipmäßig dargestellte Stromversorgung in Form einer Batterie 5 vorgesehen. Dies kann für alle Ausführungsformen gelten.

Alternativ zu der dargestellten Batterie 5 kann auch der Einsatz einer vorzugsweise auf das Bekleidungsstück 1 aufgedruckten Folienbatterie vorgesehen sein.

Anstelle oder ergänzend zu einer Batterie 5 kann auch vorgesehen sein, dass eine entsprechende Energieversorgung durch den Träger des Bekleidungsstücks 1 selbst erzeugt wird.

Die elektrischen Schaltkreise 2 kommunizieren mit einer ebenfalls in Fig. 3 dargestellten Auswerteeinheit 3, welche Signale drahtgestützt oder drahtlos an eine Rechnereinheit 6 weitergibt. Die Rechnereinheit 6 kann vorzugsweise drahtlos die Informationen an eine zentrale Überwachungseinheit, beispielsweise einen zentralen Einsatzrechner 7, weiterleiten. Zur Weiterleitung der Signale kann, wie aus Fig. 3 ersichtlich ist, eine Funkeinheit 8 und/oder eine Schnittstelle, im Ausführungsbeispiel eine USB-Schnittstelle 9, vorgesehen sein.

Die Auswerteeinheit 3 kann integraler Bestandteil der Rechnereinheit 6 sein.

Im Ausführungsbeispiel sind die USB-Schnittstelle 9 und die Funkeinheit 8 integraler Bestandteil des Bekleidungsstücks 1. Dabei kann vorgesehen sein, dass die Funkeinheit 8 die Signale direkt an eine weiter entfernte Auswerteeinheit weiterleitet oder mit einer Auswerteeinheit 3 bzw. einer Rechnereinheit 6 kommuniziert, die am Körper der Person befestigt ist, die das Bekleidungsstück 1 beispielsweise in Form eines Rucksacks trägt.

Im Ausführungsbeispiel ist vorgesehen, dass die elektrischen Schaltkreise 2 fortwährend Informationen über den Zustand des Trägers an eine Auswerteeinheit 3 und gegebenenfalls an den zentralen Einsatzrechner 7 übermitteln.

Wie sich aus Fig. 4 ergibt, ist ergänzend zu den elektrischen Schaltkreisen 2 im Ausführungsbeispiel ein Vitalsensor 10 vorgesehen, welcher den Herzschlag überwacht. Der Vitalsensor 10 ist dabei im Bereich des Herzens in das Bekleidungsstück 1 integriert.

In nicht näher dargestellter Weise kann vorgesehen sein, dass die elektrischen Schaltkreise 2 zwischen zwei Schichten angeordnet sind bzw. dass die elektrischen Schaltkreise 2 so ummantelt sind, dass eine unbeabsichtigte Beschädigung des elektrischen Netzwerks, welches die Leiterbahnen der verschiedenen elektrischen Schaltkreise 2 aufspannen, ausgeschlossen ist. Dabei kann beispielsweise vorgesehen sein, dass die Ummantelung Teil einer Beschichtung ist, welche gleichzeitig die Wasserdichtigkeit des Bekleidungsstücks 1 herstellt.

In nicht näher dargestellter Weise kann vorgesehen sein, dass die elektrischen Schaltkreise 2 in Kombination mit Piezoelementen bzw. piezoelektrischen Elementen eingesetzt werden. Die piezoelektrischen Elemente 2 und die elektrischen Schaltkreise 2 können dabei vorzugsweise in zwei Schichten vorzugsweise übereinander in dem Bekleidungsstück 1 eingesetzt sein, so dass sowohl Druckbelastungen bzw. Druckwellen als auch eine Zerstörung der Sensoren 2 durch Schuss- oder Stichwaffen erkannt wird.

In nicht näher dargestellter Weise können die Sensoren 2 sowohl in einer Ausgestaltung als elektrische Schaltkreise als auch in einer Ausgestaltung als Piezoelemente auf einer Folie angeordnet sein.

Besonders einfach lässt sich dies realisieren, wenn die Sensoren 2 als elektrische Schaltkreise ausgebildet sind. In diesem Fall können die elektrischen Schaltkreise auf die Folie gedruckt werden. In einer Ausgestaltung der Sensoren als piezoelektrische Elemente kann vorgesehen sein, dass diese als Piezofolie ausgebildet sind.

Besonders vorteilhaft kann es dabei sein, wenn die Sensoren 2 als elektrische Schaltkreise ausgebildet und direkt auf das Bekleidungsstück aufgedruckt bzw. aufgebracht sind. Dabei kann auch vorgesehen sein, dass sich die elektrischen Schaltkreise zwischen zwei Textillagen des Bekleidungsstücks 1 befinden bzw. so auf das Bekleidungsstück 1 aufgedruckt sind, dass eine versehentliche Beschädigung der Leiterbahnen der elektrischen Schaltkreise weitgehend ausgeschlossen wird.

Die in Fig. 4 dargestellten Zonen 4 sind im Ausführungsbeispiel sowohl an der Vorder- als auch an der Rückseite des Bekleidungsstücks 1 ausgebildet.

In einer zweiten Ausführungsform können die in den Figuren 1 bis 4 dargestellten Sensoren auch als Elemente 2 ausgebildet sein, deren Sensoreigenschaft auf einem Piezoeffekt beruht. Im Ausführungsbeispiel sind diese Elemente dabei als Polymere 2 ausgebildet, welche durch Polarisation piezoelektrische Eigenschaften aufweisen. Im Ausführungsbeispiel handelt es sich bei dem Polymer 2 um ein Polyvinylidenfluorid (PVDF). Das PVDF ist dabei vorzugsweise in einer Wabenstruktur bzw. mäanderförmig auf dem Bekleidungsstück 1 aufgebracht, so wie dies in den Figuren 1 bis 4 dargestellt ist.

Die vorstehend bezüglich der ersten Ausführungsform der Sensoren als elektrische Schaltkreise 2 beschriebenen Merkmale und Varianten können analog auch bei einer Ausgestaltung der Sensoren aus einem Polymer, vorzugsweise einem Polyvinylidenfluorid 2 realisiert werden.

In nicht näher dargestellter Weise ist vorgesehen, dass auf die durch das Polyvinylidenfluorid 2 gebildete Wabenstruktur ein weiterer Sensor in Form eines elektrisch leitenden bzw. leitfähigen Polymer aufgebracht wird, bei dem es sich im Ausführungsbeispiel vorzugsweise um ein Polyethylendioxythiopen (PEDOT) handelt. Das Polyethylendioxythiopen weist dabei eine Struktur aus Leiterbahnen bzw. eine Leiterstruktur auf, welche in ihrer Struktur der Struktur des elektrischen Schaltkreises 2 entsprechen kann, so wie dieser vorstehend bezüglich der ersten Ausführungsform beschrieben wurde.

Das Bekleidungsstück 1 weist vorzugsweise eine Schaltung auf, durch die festgestellt werden kann, wenn auf die Sensoren auf Basis des Polyvinylidenfluorids 2 ein Druck aufgebracht wird. In diesem Fall wird über die Leiterbahnen aus dem Polyethylendioxythiopen ein Impuls übertragen. Ferner soll die Schaltung erkennen können, ob die Leiterbahnen aus dem Polyethylendioxythiopen beschädigt bzw. unterbrochen sind - was auf einen ballistischen Einschlag bzw. einen Durchschuss oder eine Stichverletzung schließen lässt. Hierfür kann es vorteilhaft sein, wenn an die Leiterbahnen aus dem Polyethylendioxythiopen eine Spannung angelegt ist, bei deren Änderung bzw. Entfall ein entsprechendes Signal erzeugt wird bzw. eine Erkennung durch die Schaltung erfolgt. In einfacher Weise kann dies beispielsweise dadurch erfolgen, dass die Spannung, die durch die piezoelektrischen Eigenschaften des Polyvinylidenfluorids erzeugt wird, und die Spannung, die an den Leiterbahnen zur Erkennung deren Zerstörung anliegt, unterschiedliche Frequenzen aufweisen.

Die Schaltung kann ein Teil der Auswerteeinheit sein oder diese darstellen.

Von Vorteil ist es, wenn die Leiterbahnen Verbindungsstellen und/oder Lötstellen aufweisen, an denen weitere Sensoren, insbesondere Vitalsensoren, angeschlossen werden können.

Die Energieversorgung des Systems kann vorzugsweise durch aufgedruckte Folienbatterien erfolgen oder in der Art und Weise, wie dies nachfolgend bezüglich der Figuren 5 bis 7 dargestellt ist.

Ein Polymer mit piezoelektrischen Eigenschaften, wie vorstehend beschrieben, kann auch mit einem elektrischen Schaltkreis bzw. kleinen elektrischen Netzwerken kombiniert werden, so wie diese bezüglich der ersten Ausführungsform beschrieben sind.

Die Ausbildung von Piezoelementen auf Basis eines Polyvinylidenfluorids bzw. allgemein eines Polymers eignet sich auch zur Verwendung bei einer Schutzweste. Eine Schutzweste ist beispielhaft nachfolgend anhand der Figuren 5 bis 7 dargestellt. Auch eine Ausbildung von Leiterbahnen auf Basis eines Polyethylendioxythiopen bzw. allgemein eines elektrisch leitenden Polymers eignet sich zur Verwendung bei Schutzwesten.

In Fig. 5 ist prinzipmäßig eine Schutzweste 1 dargestellt, deren Aufbau aus dem allgemeinen Stand der Technik grundsätzlich bekannt ist. Die Schutzweste 1 weist eine gepanzerte bzw. mit ballistischen Schutzpaketen 11, 12 versehene Vorderseite und gegebenenfalls eine ebenso ausgebildete Rückseite auf. Die Rückseite kann dabei zusätzlich mit Trägerelementen versehen sein, um Gepäck zu transportieren, beispielsweise einen Rucksack, der auch gleichzeitig eine Rechnereinheit 6 aufweisen kann. Des weiteren kann vorgesehen sein, dass der Rucksack eine Batterie beinhaltet. Vorzugsweise ist jedoch vorgesehen, dass die Batterie Bestandteil des in den Figuren 6 und 7 näher dargestellten Schutzpakets 11, 12 ist.

Fig. 6 zeigt eine softballistische Ausgestaltung des Schutzpakets 11. Softballistische geschosshemmende Verbunde bzw. sogenannte softballistische Schutzpakete sind aus dem allgemeinen Stand der Technik hinlänglich bekannt, weshalb nachfolgend nur auf die für die Erfindung wesentlichen Merkmale näher eingegangen wird.

Der in Fig. 6 dargestellte geschosshemmende Verbund 11 ist aus mehreren miteinander verbundenen Lagen 13 eines Gewebes, vorliegend aus mehreren Lagen eines textilen Werkstoffs, gebildet. Im Ausführungsbeispiel sind 20 bis 40, vorzugsweise 28, Lagen 13 des textilen Werkstoffs vorgesehen. Bei dem textilen Werkstoff kann es sich um einen beliebigen textilen Werkstoff handeln, der geeignet ist, im Verbund die gewünschte geschosshemmende Wirkung zu erzielen und gleichzeitig flexibel bleibt. In besonderer Weise eignet sich hierfür Aramid, z. B. aromatische Polyamidfasern.

In der in Fig. 6 dargestellten Ausführungsform ist vorgesehen, dass ein Energiespeicher 14 aus mehreren Energiespeicher-Lagen 15 gebildet und in dem geschosshemmenden Verbund 11 integriert ist. Dabei sind die Energiespeicher-Lagen 15 jeweils zwischen Aramidlagen 13 angeordnet. Vorgesehen sein kann dabei, dass zwischen zwei Energiespeicher-Lagen 15 wenigstens eine Aramidlage 13 angeordnet ist. Im Ausführungsbeispiel ist vorgesehen, dass es sich hierbei genau um eine Lage 13 handelt. Im Ausführungsbeispiel sind vier Energiespeicher-Lagen 15 vorgesehen, die in dem geschosshemmenden Verbund 11 jeweils getrennt durch Aramidlagen 13 an der dem Körper zugewandten Seite angeordnet sind.

In der softballistischen Ausgestaltung des geschosshemmenden Verbunds 11 befindet sich der Energiespeicher 14 hinter dem softballistischen Schutzpaket, das heißt an der von der Außenseite abgewandten Seite des geschosshemmenden Verbunds 11. Auf dieser Seite sind auch die erfindungsgemäßen Sensoren 2 angeordnet. Im Ausführungsbeispiel kann dabei vorgesehen sein, dass die Sensoren 2 in eine hüllenförmige Folie 16, die vorzugsweise wasserdicht ist, integriert oder auf dieser angeordnet sind. Die Anordnung erfolgt dabei vorzugsweise an der dem Körper zugewandten Seite der Hülle 16. Alternativ dazu kann auch direkt in dem geschosshemmenden Verbund 11 eine Folie oder eine Lage integriert sein, die die Sensoren 2 beinhaltet. Vorzugsweise in einem an den Körper des Trägers angrenzenden Bereich, beispielsweise zwischen der letzten Energiespeicher-Lage 15 und der Außenhülle 16.

In nicht näher dargestellter Weise kann vorgesehen sein, dass der in Fig. 6 dargestellte geschosshemmende Verbund 11 mit einem zusätzlichen Stichschutz und/oder Splitterschutz versehen ist.

Die Energiespeicher-Lagen 15 können im Ausführungsbeispiel als sehr dünne Folienbatterien ausgebildet sein.

Die Sensoren 2 können dabei als druckempfindliche Sensoren, als piezoelektrische Elemente, als elektrische Schaltkreise oder kleine elektrische Netzwerke ausgebildet sein. Möglich ist auch eine Kombination der elektrischen Schaltkreise mit den piezoelektrischen Elementen bzw. den druckempfindlichen Sensoren. Von Vorteil ist es, wenn die Sensoren 2, insbesondere in einer Ausgestaltung als elektrische Leiter, auf der UV-Schutzfolie des softballistischen Schutzpakets 11 aufgebracht bzw. in diese integriert sind. Dies ist insbesondere dadurch in einfacher Weise möglich, dass die elektrischen Schaltkreise als gedruckte Schaltungen ausgebildet sind.

Fig. 7 zeigt die Ausgestaltung eines hartballistischen Schutzpakets.

Der in Fig. 7 dargestellte hartballistische Verbund 12 weist ebenfalls einen Energiespeicher 14 auf. Dieser ist ebenfalls an der dem Körper zugewandten Seite des geschosshemmenden Verbunds 12 angeordnet. D. h. der Energiespeicher 14 befindet sich in der hartballistischen Ausgestaltung hinter dem sogenannten "strike face". Der geschosshemmende Verbund 12 weist eine starre, geschosshemmende Platte 17 auf, welche mit dem Energiespeicher 14 verbunden ist. Die Platte 17 kann aus unterschiedlichsten Keramiken (vorzugsweise Hochleistungskeramiken), Polymeren, Polyethylenen, Metallen oder einer Kombination der vorgenannten Materialien bestehen. Im Ausführungsbeispiel ist die starre Platte 17 als Keramikplatte (vorzugsweise aus Borcarbid) ausgebildet. Die starre Platte 17 ist mit mehreren Lagen eines Gewebes 18 verbunden. Bei dem Gewebe handelt es sich vorzugsweise um Aramid 18 oder ein ähnliches Material. Im Ausführungsbeispiel ist die Keramikplatte 17 mit mehreren Lagen Aramid 18 verbunden, das zunächst weich bzw. flexibel als Gewebe/Gelege vorliegt, und dann mittels einer nicht näher dargestellten Klebefolie oder Klebern mit der Keramikplatte 17 unter Einwirkung von Druck und Temperatur fest, unlösbar und starr verbunden ist. Die Keramikplatte 17 ist ausgebildet, um auch sogenannten Langkerngeschossen standzuhalten.

Im Ausführungsbeispiel sind 30 bis 40, vorzugsweise 36, Aramidlagen 18 vorgesehen.

Vorgesehen ist, dass der Energiespeicher 14 wenigstens eine Energiespeicher-Lage 15 aufweist. Im Ausführungsbeispiel dargestellt ist lediglich eine Energiespeicher-Lage 15.

Gemäß Fig. 7 ist vorgesehen, dass die Energiespeicher-Lage 15 mit der Keramikplatte 17 unlösbar und starr verklebt ist.

Der geschosshemmende Verbund 12 ist von einer Außenhülle 19 umschlossen. Bei der Außenhülle 19 kann es sich um eine Folie handeln, die die Eigenschaft aufweist, die bereits bezüglich dem Ausführungsbeispiel gemäß Fig. 6 beschrieben wurde. Vorzugsweise ist die Außenhülle 19 aus einem gummierten Material gebildet, welches den Verbund 12 umgibt. Vorgesehen sein kann dabei auch eine sogenannte "Schachtellösung", bei der eine vorzugsweise starre bzw. feste Box vorgesehen ist, in welche der Verbund 12 eingelegt und die anschließend durch einen Deckel verschlossen wird. Der Verbund 12 kann in der Schachtel beliebig fixiert, vorzugsweise vergossen oder verklebt werden.

Die Anordnung der Sensoren 2 kann bei dem hartballistischen Schutzpaket in der Art und Weise erfolgen, wie dies bereits hinsichtlich der Ausführungsform gemäß Fig. 6 beschrieben wurde.

Bei der Ausführungsform gemäß den Figuren 6 und 7 kann auch auf die Anordnung eines Energiespeichers 14 bzw. die Anordnung von Energiespeicher-Lagen 15 verzichtet werden.

## Patentansprüche

1. Bekleidungsstück für einen menschlichen Körper, mit Sensoren, die externe Einwirkungen detektieren und ein Signal erzeugen, welches an eine Auswerteeinheit weiterleitbar ist, wobei die Sensoren (2) zur Druckerfassung als Elemente ausgebildet sind, deren Sensoreigenschaft auf dem Piezoeffekt beruht, wobei die Sensoren (2) wenigstens in Zonen (4) des Bekleidungsstücks (1) angeordnet sind, die an lebenswichtige Organe angrenzen, wobei in den Zonen (4) zwei Sensorentypen (2) übereinander angeordnet sind, **dadurch gekennzeichnet, dass** es sich bei einem Sensorentyp um die Elemente mit den Piezoeigenschaften und bei dem anderen Sensorentyp um einen elektrischen Schaltkreis oder ein elektrisch leitendes Polymer handelt, wobei die Elemente (2) mit den Piezoeigenschaften ein Signal erzeugen, wenn auf diese eine Druckbelastung ausgeübt wird, und wobei eine Schuss- oder Stichverletzung durch eine Unterbrechung oder Zerstörung des anderen Sensortyps, nämlich des elektrischen Schaltkreises (2) oder des elektrisch leitenden Polymers (2) registriert und an die Auswerteeinheit weitergeleitet wird.

2. Bekleidungsstück nach Anspruch 1,
**dadurch gekennzeichnet, dass**
die Sensoren (2) mehrere Zonen (4) ausbilden, die unterschiedlichen lebenswichtigen Organen zugeordnet sind.

3. Bekleidungsstück nach Anspruch 1 oder 2,
**dadurch gekennzeichnet, dass**
die Zonen (4) an der Vorderseite und der Rückseite des Bekleidungsstücks (1) ausgebildet sind.

4. Bekleidungsstück nach einem der Ansprüche 1 bis 3,
**dadurch gekennzeichnet, dass**
die Zonen (4) insgesamt so angeordnet sind, dass das Bekleidungsstück (1) vollständig abgedeckt ist.

5. Bekleidungsstück nach einem der Ansprüche 1 bis 4,
**dadurch gekennzeichnet, dass**
zur Weiterleitung eines Signals zu einer Überwachungseinheit (7) eine Funkeinheit (8) in das Bekleidungsstück (1) integriert ist oder eine Verbindung zu einer externen Funkeinheit vorgesehen ist.

6. Bekleidungsstück nach einem der Ansprüche 1 bis 5,
**dadurch gekennzeichnet, dass**
die Sensoren (2) eingestickt oder eingewebt sind.

7. Bekleidungsstück nach einem der Ansprüche 1 bis 6,
**dadurch gekennzeichnet, dass**
die elektrischen Schaltkreise (2) oder die elektrisch leitenden Polymere (2) als gedruckte Folien ausgebildet sind.

8. Bekleidungsstück nach einem der Ansprüche 1 bis 7,
**dadurch gekennzeichnet, dass**
die elektrischen Schaltkreise (2) oder die elektrisch leitenden Polymere (2) direkt auf das Bekleidungsstück (1) oder eine mit dem Bekleidungsstück (1) verbundene Textilie gedruckt sind.

9. Bekleidungsstück nach einem der Ansprüche 1 bis 8,
**dadurch gekennzeichnet, dass**
die elektrischen Schaltkreise (2) oder das elektrisch leitende Polymer (2) in Wellen- oder Kurvenform verlaufende Leiterbahnen aufweisen.

10. Bekleidungsstück nach Anspruch 1,
**dadurch gekennzeichnet, dass**
die Elemente, deren Sensoreigenschaft auf dem Piezoeffekt beruht, als Polymer (2) ausgebildet sind und das Polymer (2) ein Polyvinylidenfluorid (PVDF) ist, wobei das auf das Polyvinylidenfluorid (2) aufgebrachte elektrisch leitende Polymer (2) oder der elektrische Schaltkreis (2) in Form von Leiterbahnen aufgebracht ist.

11. Bekleidungsstück nach Anspruch 10,
**dadurch gekennzeichnet, dass**
das elektrisch leitende Polymer (2) auf Basis eines Polyethylendioxythiopen (PEDOT) gebildet ist.

12. Bekleidungsstück nach Anspruch 10 oder 11,
**dadurch gekennzeichnet, dass**
eine Schaltung vorgesehen ist, welche zum Einen über die elektrisch leitenden Polymere Impulse des Polyvinylidenfluorid (2) erkennt und zum Anderen eine Zerstörung der durch das elektrisch leitende Polymer (2) gebildeten Leiterbahnen erkennt.

13. Bekleidungsstück nach einem der Ansprüche 10 bis 12,
**dadurch gekennzeichnet, dass**
die Leiterbahnen Verbindungsstellen zur Anbindung weiterer Sensoren, vorzugsweise Vitalsensoren (10), aufweisen

14. Bekleidungsstück nach einem der Ansprüche 1 bis 13
**gekennzeichnet durch**
eine Ausbildung als soft- oder hartballistische Schutzweste (1), wobei die Sensoren (2) auf der Außenhülle (16,19) eines soft- (11) oder hartballistischen (12) Schutzpakets der Schutzweste (1) aufgebracht sind.

## Claims

1. A piece of clothing for a human body, having sensors which detect external effects and generate a signal which can be relayed to an analysis unit, wherein for sensing pressure the sensors (2) are formed as elements, the sensing properties of which are based on the piezo effect, the sensors (2) being arranged at least in zones (4) of the piece of clothing (1) which adjoin vital organs, in the zones (4) the two sensor types (2) being arranged one over another,
**characterized in that**
on sensor type is the elements having the piezo properties and the other sensor type is an electrical circuit or an electrically conductive polymer, wherein the elements (2) having the piezo properties generate a signal, if a pressure load is exerted on them and wherein a shot or stitch damage is registered by an interruption or destruction of the other sensor type, namely the electrical circuit (2) or the electrically conductive polymer (2) and is relayed to the analysis unit.

2. The piece of clothing according to Claim 1,
**characterized in that**
the sensors (2) implement multiple zones (4), which are assigned to various vital organs.

3. The piece of clothing according to Claim 1 or 2,
**characterized in that**
the zones (4) are implemented on the front side and the rear side of the piece of clothing (1).

4. The piece of clothing according to Claim 1, 2, or 3,
**characterized in that** the zones (4) are arranged as a whole so that the piece of clothing (1) is completely covered.

5. The piece of clothing according to one of Claims 1 to 4,
**characterized in that**, to relay a signal to a monitoring unit (7), a radio unit (8) is integrated in the piece of clothing (1) or a connection to an external radio unit is provided.

6. The piece of clothing according to one of Claims 1 to 5,
**characterized in that**
the sensors (2) are stitched or woven in.

7. The piece of clothing according to one of Claims 1 to 6,
**characterized in that**
the electrical circuits (2) or the electrically conductive polymers (2) are implemented as printed films.

8. The piece of clothing according to one of Claims 1 to 7,
**characterized in that**
the electrical circuits (2) or the electrically conductive polymers (2) are printed directly on the piece of clothing (1) or a textile connected to the piece of clothing (1).

9. The piece of clothing according to one of Claims 1 to 8,
**characterized in that**
the electrical circuits (2) or the electrically conductive polymer (2) have printed conductors extending in the form of waves or curves.

10. The piece of clothing according to Claim 1,
**characterized in that**
the elements (2) the sensor property of which is based on the piezo effect are formed as polymer (2) and that the polymer (2) is a polyvinylidene fluoride (PVDF), wherein the electrically conductive polymer (2) applied to the polyvinylidene fluoride or the electrical circuit (2) is applied in the form of printed conductors.

11. The piece of clothing according to Claim 10,
**characterized in that**
the electrically conductive polymer (2) is formed based on a polyethylene dioxy-thiopene (PEDOT).

12. The piece of clothing according to one of Claims 10 and 11,
**characterized in that**
a circuit is provided, which, on the one hand, recognizes pulses of the polyvinylidene fluoride (2) via the electrically conductive polymers, and, on the other hand, recognizes a destruction of the printed conductors formed by the electrically conductive polymer (2).

13. The piece of clothing according to one of Claims 10, to 12,
**characterized in that**
the printed conductors have connection points for attaching further sensors, preferably vital sensors (10).

14. The piece of clothing according to one of Claims 1 to 13,
**characterized by**
an implementation as a soft-ballistic or hard-ballistic protective vest (1), wherein the sensors (2) are applied to the external sheath (16,19) of a soft-ballistic (11) or hard-ballistic (12) protective packet of the protective vest (1).

## Revendications

1. Pièce de vêtement pour un corps humain, avec des capteurs qui détectent un signal qui peut être retransmis à une unité de traitement, dans lequel les capteurs (2) sont conçus comme des éléments qui sont sensibles aux pressions, et dont les caractéristiques de détection sont basées sur l'effet piézoélectrique, dans lequel les capteurs (2), sont disposés au moins dans des zones (4) de la pièce de vêtement (1) qui correspondent à des organes vitaux essentiels, dans lequel, dans les zones (4), deux capteurs (2) sont superposés, **caractérisée en ce que** pour un type de capteurs, il s'agit des éléments ayant des caractéristiques piézoélectriques et pour l'autre type de capteurs, d'un circuit électrique de commande ou d'un polymère conducteur, dans lequel les éléments (2) avec les caractéristiques piézoélectriques génèrent un signal, lorsqu'une contrainte de pression est exercée sur eux, et dans lequel, lorsqu'une blessure ou une interruption de la trame ou de la chaîne sous la forme d'une rupture ou d'une perturbation de l'autre type de capteur est enregistrée, en fait du circuit électrique (2) ou du polymère conducteur (2) et est communiqué à l'unité de traitement.

2. Pièce de vêtement selon la revendication 1, **caractérisée en ce que** les capteurs (2) sont réparties dans différentes zones (4) qui correspondent respectivement à divers organes d'importance vitale.

3. Pièce de vêtement selon la revendication 1 ou 2, **caractérisée en ce que** les zones (4) sont disposées sur la partie avant et sur la partie arrière de la pièce de vêtement (1).

4. Pièce de vêtement selon l'une des revendications 1 à 3, **caractérisée en ce que** les zones (4) sont disposées de telle manière que la pièce de vêtement (1) soit entièrement recouverte.

5. Pièce de vêtement selon l'une des revendications 1 à 4, **caractérisée en ce que**, pour la transmission d'un signal vers une unité de surveillance (7), une station radio (8) est intégrée dans la pièce de vêtement (1) ou une liaison de communication vers une station extérieure est prévue.

6. Pièce de vêtement selon l'une des revendications 1 à 5, **caractérisée en ce que** les capteurs (2) sont intégrés sous forme de broderie ou par tissage.

7. Pièce de vêtement selon l'une des revendications 1 à 6, **caractérisée en ce que** les circuits électriques (2) ou les polymères (2) électriquement conducteurs sont réalisés sous forme de feuilles imprimées.

8. Pièce de vêtement selon l'une des revendications 1 à 7, **caractérisée en ce que** les circuits électriques (2) ou les polymères (2) électriquement conducteurs sont directement imprimés sur la pièce de vêtement (1) ou sont imprimés sur des textiles liés à la pièce de vêtement (1).

9. Pièce de vêtement selon l'une des revendications 1 à 8, **caractérisée en ce que** les circuits électriques (2) ou les polymères (2) électriquement conducteurs comportent des pistes conductrices sous forme de vagues ou de lignes courbes.

10. Pièce de vêtement selon la revendication 1, **caractérisée en ce que** les éléments avec les caractéristiques de capteurs basés sur l'effet piézoélectrique, sont réalisés sous forme de polymère (2) et le polymère (2) est un fluorite de polyvinyle (PVDF), dans lequel le polymère (2) électriquement conducteur fixé sur le fluorite de polyvinyle (2) ou le circuit électrique (2) est constitué de bandes conductrices.

11. Pièce de vêtement selon la revendication 10, **caractérisée en ce que** le polymère électriquement conducteur (2) est formé sur la base d'un dioxythyopène de polyéthylène (PEDOT).

12. Pièce de vêtement selon la revendication 10 ou 11, **caractérisée en ce qu'**un circuit électrique est prévu, d'une part pour reconnaître des impulsions du fluorite de polyvinyle (2) à travers le polymère électriquement conducteur et, d'autre part identifier un dérangement des pistes conductrices constituées par le polymère électriquement conducteur (2).

13. Pièce de vêtement selon l'une des revendications 10 à 12, **caractérisée en ce que** les pistes conductrices comportent des zones de connexion pour assurer le couplage de capteurs complémentaires, de préférence des capteurs vitaux (10).

14. Pièce de vêtement selon l'une des revendications 1 à 13, **caractérisée par** une utilisation comme veste de protection balistique (1) légère ou renforcée, dans lequel les capteurs (2) sont disposés sur la paroi extérieure (16, 19) d'un bloc de protection balistique léger (11) ou renforcé (12) ou d'une veste de protection (1).
